Europäisches Patentamt

**European Patent Office**

Office européen des brevets

(11) Publication number: **0 000 670**
A1

## EUROPEAN PATENT APPLICATION

(21) Application number: 78300225.6

(22) Date of filing: 01.08.78

(51) Int. Cl.²: **A 61 K 33/04**, A 61 K 9/06,
A 61 K 31/19 //(A61K33/04,
31/375), (A61K33/04,
31/57), (A61K37/48,
33/04)

(30) Priority: 02.08.77 US 821156

(43) Date of publication of application:
07.02.79 Bulletin 79/3

(84) Designated contracting states:
BE CH DE FR GB NL SE

(71) Applicant: LEVITT RESEARCH LABORATORIES, INC.
166 East 34th Street New York
N.Y. 10016. (US)

(72) Inventor: Levitt, Joseph R.
166 East 34th Street New York
N.Y. 10016. (US)

(74) Representative: Cook, Anthony John et al
D. YOUNG & CO.
9 & 10, Staple Inn
London, WC1V 7RD. (GB)

(54) Therapeutic selenium compositions and the use thereof.

㊏ Therapeutic compositions comprising certain selenium-containing compounds which exhibit therapeutic benefits in mammal hosts including humans are disclosed. The selenium compounds in accordance with this invention are preferably water soluble organic or inorganic compounds containing selenium such as alkali metal selenites and selenates. Among the therapeutic benefits exhibited by these compositions are the reduction in severity of, and improvements in recovery from, physically induced damage to body tissue which includes damage caused by surgical incisions, lacerations and burns.

EP 0 000 670 A1

1.

## THERAPEUTIC SELENIUM COMPOSITIONS AND THE USE THEREOF

This invention relates to therapeutic compositions containing certain selenium compounds. These therapeutic compositions have been found to exhibit surprising and unexpected properties in mammalian hosts. More particularly, the selenium compounds in accordance with the present invention are water soluble organic or inorganic compounds containing selenium in a form capable of being absorbed by the body tissue to be treated. Preferably, the compounds are salts containing selenium in the form of the selenite or selenate anions. In the practice of the present invention, the therapeutic compositions are formulated so that said selenium compounds are present in certain specific amounts and concentrations so that they are non-toxic yet therapeutically active.

Selenium is one of numerous elements found in trace amounts in many foods. The selenium is present in many chemicals and often in very complex forms. Many technical studies have been carried out concerning its biological effects although the majority of the work has dealt with those adverse effects which occur by ingesting more than trace amounts of selenium-containing compounds.

Research studies to date have indicated that selenium may have beneficial physiological effects on mammals. For example, it has been suggested that selenium, when ingested, reduces the rate of oxidative damage caused by chemicals, such as, for example, ozone in smog, by entering the membrances of the body's cells and protecting

2.

the contents of the cells from reacting with oxygen in a manner that damages the cells. It has also been reported that selenium may be beneficial in the treatment of heart disease by reducing or decreasing coronary vascular resistance in dogs. Other studies have suggested that some beneficial effects may arise from the use of selenium in cancer therapy.

On the other hand, there has been considerable reluctance to prescribe usages of selenium because of its apparent toxic effects when present in large dosages. While numerous medical and governmental reports have found selenium to have general toxic properties in adult mammals, there is no consensus as to specific toxicity levels nor toxicity effects.

It is known, however, that deleterious effects on the heart, lungs, liver and kidneys do occur, in addition to adverse effects on other body systems in mammals, both humans and animals, in cases where selenium is ingested. But the particular amounts ingested which cause such effects vary widely depending upon the form of the ingested selenium, and the presence of other materials in the diet of the host. For example, protein is reported to afford some protection against the toxic effects of selenium.

It is therefore surprising that in accordance with the present invention it has been discovered that pharmaceutically safe compositions have been formulated which have unexpected therapeutic and physiological properties and benefits which enable them to be easily formulated and administered to mammals, including humans. Also provided by this invention are methods of treating mammals, including humans, to reduce the severity from and to improve recovery from certain physically induced injuries to tissue caused by physical effects or irritating stimuli such as surgical operations, lacerations and burns including electrical, sun and thermal burns, etcetera.

3.

In accordance with the present invention, there is provided an organic or inorganic water soluble compound containing selenium wherein the selenium is in such a form as to be capable of being absorbed by the tissue of the host being treated, for use in treating tissue affected by one or more of incisions, lacerations, burns, infection, edema, ecchymosis, or for minimising scarring, or for treating eye redness and irritation.

The selenium compound is preferably a water soluble organic or inorganic selenite or selenate such as alkali metal selenites or selenates. The composition may be formulated for oral, injectable, topical or suppository administration. For example, tablets capsules, solutions, creams and the like may be made where the selenium compound is dispersed substantially homogeneously throughout a suitable pharmaceutically acceptable carrier or vehicle in such finished dosage form.

Compositions in accordance with the present invention which contain certain selenium compounds in therapeutically effective but non-toxic dosages can be administered to mammals for the purpose of reducing the severity from and improving the recovery from physically induced damage to the host's tissue. By physically induced damage is meant various types of damage to the tissue of a mammal including, for example, surgical incisions, lacerations, burns, etcetera. Accompanying such damage are the likelihood of infection, edema, ecchymosis and other inflammatory responses. It has been found that where the therapeutic compositions of the present invention are administered prior to such physically induced damage to tissue, such as prior to surgery, an observable improvement in the patient's healing of the tissue and a reduced degree of infection, edema, ecchymosis and other inflammatory responses are noted.

It has also been found that the therapeutic composition of the present invention can be administered subsequent to the physically

4.

induced damage to tissue and improve the recovery of the tissue and affected areas. For example, the compositions of the present invention may be applied topically to the specifically affected areas of the skin to reduce scarring, etcetera.

The words "physically induced damage" when used in this Specification are intended to include damage due to burns. It has been found that lesions and scars to the skin due to burning and other tissue damage is reduced and in some cases eliminated and that recovery is accelerated by use of the composition of the present invention.

The anti-inflammatory properties of the selenium compositions of this invention provide useful advantages when employed alone or with other materials utilized, for example, in pre-operative or post-operative surgical procedures. For example, various compounds such as steroids have been alleged to possess anti-inflammatory properties. Included among these are, for example, cortisone and hydro-cortisone.

The selenium compositions of this invention used alone exhibit effective anti-inflammatory properties without the detrimental side effects of some known materials which exhibit anti-inflammatory properties. The selenium compounds of the present invention may be combined with materials having compatible properties to form compositions exhibiting the beneficial effects of the selenium compounds, as well as enhanced beneficial effects of these other materials.

The primary active ingredient in the therapeutic compositions of the present invention is the selenium compound or compounds. It is to be noted that selenium occurs in a number varying valence forms. For example, selenium compounds in which the selenium has a +4 valence, usually as the selenite and selenate ions, may be utilized in the compositions of this invention. Among the
selenite and selenate forms, the preferred compounds utilized
in the compositions of this invention are the water soluble alkali
metal salts thereof, and particularly, the sodium and potassium
salts, that is, sodium and potassium selenite and selenate. On
the other hand, organic compounds of selenium may also be utilized
in the compositions of this invention. For example, selenium
compounds of cystine and methionine, as well as the aliphatic
mono- and di-selenoidi-carboxylic acids having about 7 to 11
carbons in the carbon chain may be used. Particularly useful
acids of this group include monoseleno-11, 11' - di'n-undecanoic
acid, diseleno-4, 4' -di-n-valeric acid and diseleno-11, 11'-di-n-
undecanamide. It is to be understood, however, that the particular
organic forms of selenium compounds set forth herein are not to be
considered limitative. Other organic selenium compounds which
exhibit the desired activity and are compatible with the host and
non-toxic to the host can be used in the practice of this invention.

It is a critical aspect of this invention that the selenium
in the form present in the composition be capable of being absorbed
by the tissue of the body to be treated. It is noted that water
insoluble selenium compounds are not generally absorbed by the
tissue.

The compositions of this invention can be made by mixing a
suitable appropriate carrier and the selenium compound together
and agitating the mixture until homogeneity is attained.

The concentration of the selenium compound to be present in a
particular composition will depend upon the means of administraticn
and nature of the composition. For example, compositions for
topical administration should have generally a lower concentraticn
of elemental selenium than compositions for other types of
administration.

The selenium compound can be present in such an amount so that the elemental selenium concentration of the therapeutic composition is in the order of from about 0.005 to about 2 mg per gram of the total weight of the composition. The concentration of course, depends upon the therapeutic activity desired and toxicity maximum levels.

As the daily dosage range is of the order of about 0.05 to 1.0 mg and preferably 0.5 to about 1.0 elemental selenium, it is convenient as a practical matter to formulate the composition so that about 0.5 mg to about 1.0 mg of elemental selenium is present in a given dosage amount of the composition.

The compositions of the present invention are quite stable and can be pre-mixed in the form of subsequent administration. Techniques that are recognised in the art for packaging, storing and preparing medicinal compositions for administration are generally applicable to these compositions.

As noted above, the therapeutic compositions in accordance with the present invention may be made in various physical forms for well known methods of administration. For example, the composition can be in a suitable form for injectable, topical, suppository and oral administration. The choice of the particular carrier or vehicle and other additives present will depend upon the form desired. Said compositions may also be combined with other materials such as cleansing and antiseptic-type agents.

In practicing this invention, a wide variety of carriers or vehicles for the selenium can be utilized and the terminology "pharmaceutically acceptable carrier or vehicle" as employed throughout this specification and in the appended claims is to be understood to include any known carriers or vehicles generally employed in the pharmaceutical field including inert and active carriers or vehicles.  The vehicles or carriers should not detrimentally affect any of the selenium compounds of the compostion.

Exemplary inert carriers or vehicles include:  sugars and milk sugars, such as lactose;  liquids, such as water, isotonic aqueous solutions, saline solutions and alcohol;  and inert powders, creams, salves, ointments, cleansing and antiseptic agents and the like.

Pharmaceutically active carriers or vehicles may also be used. These may include physiologically active powders, liquids, salves, creams and ointments as well as materials such as vitamins, steroids including cortisone and hydrocortisone.  Other materials include proteolytic enzymes, such as those obtained from the pineapple plant and sold under the trade name of Ananase by William H. Rorer, Inc. of Fort Washington, Pa., U.S.A., proteolytic enzymes obtained from the papaya plant and sold under the trade name of Papase by Warner/Chilcott, Division of Warner-Lambert Company of Morris Plains, New Jersey, U.S.A., and others, such as animal pancreas extracts which contain trypsin and chymotrypsin, one form of such extract being sold under the trade name of Chymoral by Armour Pharmaceutical Co. of Phoenix, Arizona, U.S.A.

For oral adminstration, the therapeutic composition of the present invention can be prepared in dosage form as capsules, tablets, powders, syrups, oral solutions and the like.  These orally administrable compositions may contain such additives as diluents, fillers, lubricants and glidants.  Where they are in the form of a liquid, they should be suitably prepared so that the ingredients including the liquid pharmaceutical carrier are bland to the gastric mucosa.

In the case of the injectable form of the composition, the therapeutic selenium compound may be dissolved in distilled or sterilized water to form a parenteral preparation, or it can be mixed with intravenous infusions such as glucose or saline.

In accordance with this invention, selenium can be utilized in compositions suitable for topical, dermatological applications. In this regard, the selenium compounds can be used for skin treatments and maintenance in dermatological creams, salves, and the like. Such compositions can be effective in treating skin irritations, certain rashes, dermatitis, eczema, acne, and pruritus. Such compositions also have properties making them useful in reducing the rate of cell aging due to exposure to light and oxygen which generally results in an oxidizing condition that weakens the cell membranes, thus causing the cells to deform. In this regard, it is to be noted that the number of damaged cells generally increases rapidly with the time of exposure to light and oxygen. However, the use of dermatological preparations containing selenium in accordance with this invention dramatically interrupts or stops such damaging effects.

The topical forms of the compositions of the present invention are also particularly effective in improving and accelerating the healing of tissue damaged by burns, which includes burns caused by the sun, heat, electricity, radioactive exposure and chemicals.

It is also to be noted that selenium compositions in accordance with the invention prevent distortion or adverse bio-chemical effects in cells which are subjected to ultraviolet light which greatly damages the membrance of such cells.

The composition of the present invention may be administered as eye drops in the form of a very dilute isotonic aqueous solution, that is, from about 0.1 mg to 0.5 mg of elemental selenium per cc solution and properly buffered to substantially neutral pH of about 6-7. Such eye drop solutions are useful in relieving eye irritation and redness caused by dust, smoke, smog, wind and sun glare, swimming, strain due to reading and television viewing or in the problems encountered in adapting to the utilization of contact lenses. A daily dosage of such an eye drop solution would be in the order of 1/20 to 1/5 cc.

It is to be understood that the particular carriers or vehicles set out above are illustrative only and other known pharmaceutically acceptable materials can be utilized in the compositions of this invention so long as they do not react with the selenium and other active ingredients if any to destroy the identitiy thereof. Moreover, the particular carrier or vehicle chosen for use will depend upon the form of the composition needed for the particular method of adminstration and the host to receive the composition.

In those cases where the composition contains more than about 1.0 mg by weight, the composition may be employed in the form of divided dosages when being administered whether it be in the form of a tablet, a capsule or a liquid solution. Moreover, a particular dosage in this respect can be adminstered several times a day so long as the total amount of selenium compound does not exceed a maximum of about 1.0 mg per day.

In some instances, the composition of this invention can be made by simply mixing the selenium compound in proper proportion with an appropriate carrier. For example, in preparing tablets, an alkali metal selenite or selenate salt in its dry form may be mechanically mixed with a powdered carrier or vehicle and shaped or pressed into tablets or encapsulated by known and art-recognized techniques. On the other hand, if desirable, such salts can be dissolved in water and then mixed with a powdered carrier and shaped or pressed into tablets.

As an alternative, liquid compositions can be prepared simply by dissolving the selenium compounds in water and using the composition in that form with recognized additives for either

10.

external or oral application. The materials as mixed should contain at least a 0.05 mg dosage of selenium to achieve most therapeutic benefits and may contain up to an amount conveniently combinable with the carrier to provide a daily dosage of selenium in a range of from about .05 mg to about 1.0 mg, and preferably from about 0.5 mg to about 1.0 mg.

While the stated range is generally a beneficially useful amount of selenium in accordance with this invention, it is also to be understood that this range is normally the amount given to an adult mammalian individual. However, dependent upon the weight and age of an adult mammalian host, the amount ingested by such a host may be adjusted accordingly within the stated range or somewhat outside the minimum and maximum thereof. The total overall amount to be ingested per day by a relatively small or young individual, for example, is preferably near the lower end of the range and for larger or older individuals, toward or in the area of the upper end of the stated range. A general range for daily treatment dosages, again based upon the purpose of treatment and other factors of the individual, would be in the range 0.005 - 0.02 mg per kg weight of the host.

In using the selenium compositions of this invention with the view to obtaining the pre-operative and post-operative benefits thereof, the compositions may simply be administered orally to an adult patient or host in divided doses for several days, e.g., from 1-7 days, preceding surgery in the stated concentrations or for several days, e.g., from 1-7 days following the surgical procedure or other trauma. For example, an aqueous solution of sodium selenate or sodium selenite can be made in a concentration to provide 5.0 mg of selenium and then sub-divided by dilution to provide an equivalent amount of selenium of 0.5 to 1 mg and administered in daily amounts orally or otherwise for a period of

11.

1-5 days preceding and following surgery. On the other hand, the selenium may also be administered orally, intraveneously or subcutaneously, for example, by dissolving sodium selenate or sodium selenite in water in an amount such that one drop of the solution yields 0.33 mg of selenium. The concentration may be based upon one drop of the solution being equal to 1/20 of a ml. More dilute and concentrated solutions may also be used.

In accordance with the present invention, the effectiveness of selenium is generally enhanced by administering the same simultaneously with other physiologically active materials. For example, compositions in accordance with this invention can comprise small amounts of selenium combined with Vitamin E, wherein the Vitamin E is present in the range from about 10 I.U. to about 1000 I.U., and preferably from about 200 I.U. to about 800 I.U. The combination permits advantageous beneficial effects due to the presence of selenium with the vitamin, it being understood that in use further appropriate dilution takes place so that the daily amount of elemental selenium administered does not exceed 1.0 mg.

The selenium can also be combined with ascorbic acid, i.e., Vitamin C and utilized in the pre-treatment of patients undergoing elective surgery. Due to the fact that some selenium does form a precipitate with the Vitamin C, an appropriate method of administration should be used to minimize this precipitation effect.

The present invention presents many advantages both in its therapeutic effects and in the ability to formulate adminstrable compositions. When ingested, selenium is distributed to all of the cells in the body and is stored non-selectively but not uniformly, as is the case with several other elements such as chromium, iodine, and calcium.

Compositions of this invention are relatively simple to prepare. The water soluble sodium and potassium salts of selenium are readily

12.

available are are easily incorporated into a suitable pharmaceutical carrier or vehicle without the need for extraordinary and elaborate manufacturing equipment. Moreover, if desirable, the selenium salts can be distributed in a suitable carrier and stored for long periods of time without loss of effectiveness. On the other hand, if desired, the salts can be marketed separately and simply mixed or compounded with a carrier just prior to use. Numerous other advantages of this invention will be readily apparent to those skilled in the art.

Numerous modifications of this invention may be made without departing from the spirit and scope thereof. Accordingly, it is to be understood that this invention is not to be limited to the illustrative embodiments set forth herein.

## EXAMPLE 1

This example is presented to illustrate the tolerance and effect of compositions of the present invention as administered to humans. The patient chosen for this example was 22 years old. Some four years prior to receiving the treatment in accordance with the present invention, he had undergone a rhinoplasty surgery which resulted in severe and alarming post-operative ecchymosis, edema and blood red eyes. The rhinoplasty surgical procedure involved the fracture of the patient's nose and associated trauma associated with the surrounding tissues. This prior treatment and recovery of the patient acts as a control to the instant example.

Four years after the aforementioned rhinoplasty surgery and after some three and a half years following complete recovery, the patient received the treatment in accordance with the present invention which contained 0.65 mg of selenium. This oral dosage was prepared as follows:

13.

One ounce of sodium selenite as obtained
from City Chemical Corp., of New York,
New York, was added to ordinary tap water
to yield a concentration of 28.35 mg of
sodium selenite $(Na_2SeO_3)$ to 1 cc of
water.   This is equivalent to 13.0 mg
of elemental selenium per cc of water.

The foregoing solution was ingested in an amount daily to · yield a daily dosage of 0.65 mg per day.   The daily dosage was continued for a period of seven days.   On the evening of the seventh day of treatment, the patient was involved in an automobile accident in which he suffered head, nose and facial injuries. This included a fracture of his nose and associated trauma to the surrounding tissue.   The patient received emergency hospital treatment including examination and treatment by a plastic surgeon. During the course of the patient's recovery, there was no observed ecchymosis or edema of the nose, eyes or face, although trauma had been sustained as a result of the accident.

## EXAMPLE 2

This example illustrates the effect of orally ingesting the therapeutic composition of the present invention on the irritation of the eyes of a human patient.   The patient was a wearer of contact lenses and for many years suffered regular inflammation and irritation due to the wearing of contact lenses.   The patient received by oral ingestion the therapeutic composition prepared in accordance with Example 1 in an amount equivalent to a daily dosage of 0.5 mg of selenium.   After a period of 5 days, the redness and inflammation that was normally present in his eyes subsided and discontinued.   After his eyes reached an optimum improvement, he discontinued the ingestion of the therapeutic composition for a period of seven days.   After those seven days, the patient's eyes became inflamed, red and irritated to the same extent as existed prior to the original treatment with the

14.

therapeutic composition as noted above.    A subsequent resumption
in the ingestion of the therapeutic composition again improved
the patient's eyes as during the prior treatment period.    No side
effects from this treatment have been observed.

1.

CLAIMS

1.   An organic or inorganic water soluble compound containing
selenium wherein the selenium is in such a form as to be capable
of being absorbed by the tissue of the host being treated, for use
in treating tissue affected by one or more of incisions, lacerations,
burns, infection, edema, ecchymosis, or for minimising scarring,
or for treating eye redness and irritation.

2.   A water soluble alkali metal selenium salt for use in treating
tissue affected by one or more of incisions, lacerations, burns,
infection, edema, eccymosis, or for minimising scarring, or for
treating eye redness and irritation.

3.   A water soluble alkali metal selenite or selenate for use in
treating tissue affected by one or more of incisions, lacerations,
burns, infection, edema, eccymosis, or for minimising scarring,
or for treating eye redness and irritation.

4.   A therapeutic composition for administration to humans and
other animals for the purpose of reducing the severity from and
accelerating the recovery from physically induced damage to body
tissue, said composition being characterised in that it comprises
a water soluble organic or water soluble inorganic therapeutic
compound containing selenium wherein the selenium is in such a form
as to be capable of being absorbed by the tissue of the host being

2.

treated together with a non-toxic pharmaceutically acceptable carrier or diluent therefor, the therapeutic composition being formulated so as to provide the equivalent of 0.05 mg - 1.0 mg of elemental selenium per day to the host in single or multiple dose form.

5. The therapeutic composition as defined in claim 4 wherein the selenium is present in the form of a water soluble alkali metal salt thereof.

6. The therapeutic composition of claim 4 wherein said therapeutic compound is selected from the group consisting of alkali metal selenates and alkali metal selenites.

7. The therapeutic composition of claim 4 wherein the therapeutic compound is an organic selenium-containing compound.

8. A therapeutic composition in the form of a topical preparation and according to any of claims 4 - 7 which contains an ingredient selected from: perfumes, emollients, emulsifying agents, stabilizers, and physiologically acceptable coloring agents.

9. A therapeutic composition according to any of claims 4 - 7 and in the form of a capsule, wherein said capsule contains a material selected from the group consisting of colloidal silica, magnesium stearate, calcium phosphate, calcium sulphate, lactose, mannitol, sodium stearyl fumarate and mixtures thereof.

10. A therapeutic composition according to any of claims 4 - 7 further comprising Vitamin C or ascorbic acid.

11. A therapeutic composition according to any of claims 4 - 7 which contains a steriod.

12. A therapeutic composition according to any of claims 4 - 7 which contains a proteolytic enzyme.

3.

13. The therapeutic composition of claim 12 wherein said proteolytic enzyme is selected from the group consisting of extracts from pineapple plants, extracts from papaya plants, and extracts containing trypsin and chymotrypsin.

14. The therapeutic composition of claim 13 wherein said proteolytic enzyme is selected from Ananase and Papase.

15. The therapeutic composition of claim 4 further comprising Chymoral.

16. A cleansing composition having therapeutic properties in improving the healing and maintaining of skin tissue, said composition containing a cleansing agent and a therapeutic composition according to claim 1.

17. An eye drop solution for alleviating irritation and redness in the eyes of humans as well as other animals, said solution being characterised in that it comprises a water soluble organic or inorganic therapeutic compound containing selenium whereby the selenium is in such a form as to be capable of being absorbed, by the tissue of the eye being treated, said compound being dissolved in a suitably buffered isotonic aqueous solution in such a manner that the concentration of the compound in solution is in the range of about 0.1 mg to 0.5 mg of elemental selenium per ml. of solution.

18. A method of alleviating irritation and redness in an eye of a mammal animal, said method being characterised by administering to the eye of the animal from 1/20 ml. to 1/5 ml. of an eye drop solution in accordance with claim 17.

0000670

<table>
<tr><td colspan="2"><b>European Patent Office</b></td><td><b>EUROPEAN SEARCH REPORT</b></td><td>Application number<br>EP 78 30 0225</td></tr>
</table>

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |

| Category | Citation of document with indication, where appropriate, of relevant passages | Relevant to claim |
|---|---|---|
| | US – A – 3 928 578 (HENRY C.BURNS) <br> * Claim 1 * <br> --- | 1-6 |
| | FR – A – 2 263 780 (PEPRO) <br> * Claims 1-3 * <br> --- | 1-6 |
| | GB – A – 1 444 024 (RICHARD ALBERT PASSWATER) <br> * Claim 1 * <br> --- | 1-7,10 |
| | CHEMICAL ABSTRACTS, 84, 69541d and 69542e (1976) mentions "Selen Biol. Mater Nauchn.Konf.", 1974, 122-5, 182-92, 126-8. <br> * Abstracts * <br> --- | 1-6 |
| | CHEMICAL ABSTRACTS, 62, 13729fh (1965) mentions "J.Bacteriol." 90(4), 857-62 (1965) <br> * Abstract * <br> ---- | 1-6 |

**CLASSIFICATION OF THE APPLICATION (Int. Cl.²)**

A 61 K 33/04
A 61 K 9/06B
A 61 K 31/19
//(A 61 K 33/04
A 61 K 31/375)
//(A 61 K 33/04
A 61 K 31/57)
//(A 61 K 37/48
A 61 K 33/04)
A 61 K 37/48
//(A 61 K 31/375
A 61 K 31/19)
//(A 61 K 31/57
A 61 K 31/19)

./.

**TECHNICAL FIELDS SEARCHED (Int.Cl.²)**

A 61 K 33/04
A 61 K 9/06B
A 61 K 31/19
A 61 K 37/48
A 61 K 31/375
A 61 K 31/57

**CATEGORY OF CITED DOCUMENTS**

X: partioularly relevant
A: technological background
O: non-written disclosure
P: intermediate document
T: theory or principle underlying the invention
E: conflicting application
D: document cited in the application
L: citation for other reasons

&: member of the same patent family, corresponding document

| X | The present search report has been drawn up for all claims | | |
|---|---|---|---|
| Place of search <br> The Hague | Date of completion of the search <br> 2-11-1978 | Examiner <br> BRINKMANN | |

EPO Form 1503.1 06.78

0000670

## DOCUMENTS CONSIDERED TO BE RELEVANT

| Category | Citation of document with indication, where appropriate, of relevant passages | Relevant to claim |
|---|---|---|
|  |  |  |

**CLASSIFICATION OF THE APPLICATION (Int. Cl.²)**

// (A 61 K 37/48
A 61 K 31/19)
A 61 K 31/375
A 61 K 31/57

**TECHNICAL FIELDS SEARCHED (Int. Cl.²)**